# EUROPÄISCHE PATENTSCHRIFT

(11) **EP 1 663 964 B1**
(45) Veröffentlichungstag und Bekanntmachung des Hinweises auf die Patenterteilung: **02.07.2008**
(21) Anmeldenummer: 04765052.8
(22) Anmeldetag: 10.09.2004
(51) Int. Cl.: C07C 323/19, C07D 301/12

(54) **VERFAHREN ZUR HERSTELLUNG VON BISEPOXIDEN UND DITHIOLEN**
METHOD FOR THE PRODUCTION OF BISEPOXIDES AND DITHIOLS
PROCEDE POUR PRODUIRE DES BISEPOXYDES ET DES DITHIOLS

(30) Priorität: 17.09.2003 DE 10343252
(43) Veröffentlichungstag der Anmeldung: 07.06.2006
(60) Teilanmeldung: 07104119.8
(73) Patentinhaber: BASF SE, 67056 Ludwigshafen (DE)
(72) Erfinder: BÜHLER, Holger, 67122 Altrip (DE); TELES, Joaquim, Henrique, 67166 Otterstadt (DE); PABST, Gunther, 92318 Neumarkt (DE); TAEGER, Tilman, Lüdecke, 64342 Seeheim-Jugenheim (DE)
(86) Internationale Anmeldenummer: PCT/EP2004/010123
(87) Internationale Veröffentlichungsnummer: WO 2005/033070

(56) Entgegenhaltungen:
- DE-A1- 2 131 630
- DE VOS D E ET AL: "Epoxidation of Terminal or Electron-deficient Olefins with H2O2, catalysed by Mn-trimethyltriazacyclonane Complexes in the Presence of an Oxalate Buffer" TETRAHEDRON LETTERS, ELSEVIER SCIENCE PUBLISHERS, AMSTERDAM, NL, Bd. 39, Nr. 20, 14. Mai 1998 (1998-05-14), Seiten 3221-3224, XP004116236 ISSN: 0040-4039 in der Anmeldung erwähnt

## Beschreibung

Die vorliegende Erfindung betrifft ein Verfahren zur Herstellung von Bisepoxiden, dadurch gekennzeichnet, dass man ein konjugiertes Dien der allgemeinen Formel I 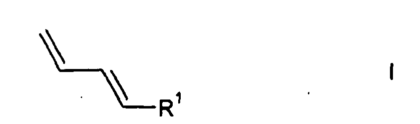 in dem
- R¹: gewählt wird aus Wasserstoff und C₁-C₁₂-Alkyl, unsubstituiert oder substituiert mit einer oder mehreren S-H oder O-H-Gruppen,
in Gegenwart eines Katalysators, der erhältlich ist durch Kontaktieren von
mindestens einer Manganverbindung, gewählt aus A₂MnX₄, AMnX₃, MnY, MnX₂ und MnX₃
mit mindestens einem Liganden L der allgemeinen Formel II 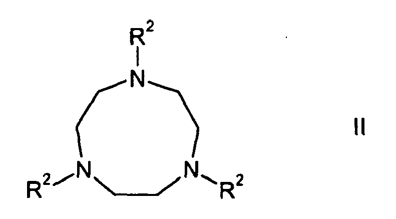
wobei die Variablen wie folgt definiert sind:
- X: verschieden oder gleich und gewählt aus einwertigen Anionen,
- Y: ein zweiwertiges Anion,
- A: gewählt aus Alkalimetall und Ammonium, welches alkyliert sein kann,
- R²: verschieden sind oder vorzugsweise gleich und gewählt aus C₁-C₂₀-Alkyl, und mindestens einem Coliganden, der von Monocarbonsäuren, zwei- oder mehrwertigen Carbonsäuren oder Diaminen abgeleitet ist,
mit mindestens einem Peroxid umsetzt, wobei man bis zu 4 Äquivalenten Peroxid pro Äquivalent C-C-Doppelbindung einsetzt.

Dithiole sind äußerst vielseitige Reagenzien. So ist bekannt, dass man 1,4-Dimercaptobutandiol bei der Aufreinigung und Stabilisierung von Enzymen (Schutzreagens für - SH Gruppen, W.W. Cleland, Biochemistry, 3, 480, 1964) einsetzen kann. Weiterhin ist aus DE 22 09 458 bekannt, dass sogenanntes 1,4-Dithiol-2,3-butandiol und seine Metallsalze als Haarwellmittel und Enthaarungsmittel verwendet werden können. Aus DE 23 37 101 ist bekannt, dass man n-Butan-2,3-diol-1,4-dithiol und seine Alkalimetallsalze zur Erzeugung von Dauerwellen verwenden kann. Aus DE 21 31 630 ist bekannt, dass man Mittel, bestehend aus mindestens 0,25 Gew.-% Dimercaptobutandiol und etwa 0,01 bis 40 Gew.-% einer wasserlöslichen Guanidinverbindung und einem pH-Wert von unter 12 auf Meerschweinchen aufbringen kann, um sie zu enthaaren, oder auf menschliche Hornhaut, um Schwielen zu beseitigen, ohne dass es zu Hautreizungen bei Meerschweinchen oder gar zu Erythrämie (bösartige Wucherungen des Bildungssystems der roten Blutkörperchen) kommt. Die Epidermis bleibt bei der in DE 21 31 630 beschriebenen Behandlung erhalten.

Es ist also wünschenswert, eine Syntheseroute zu finden, durch die man n-Butan-2,3-diol-1,4-dithiol und Derivate mit guter Ausbeute und in guter Reinheit gewinnen kann.

Aus DE 22 09 458 und DE 23 37 101 ist bekannt, dass man racemisches Dithiolbutandiol, verunreinigt mit geringfügigen Mengen an Dithioerythrol, herstellen kann, indem man Butadienbisepoxid mit Schwefelwasserstoff in Gegenwart eines alkalischen Katalysators im Bereich zwischen 15 und 40°C in einem Schwefelwasserstoff lösenden Mittel umsetzt, dessen Volumen zum Volumen an Butadienbisepoxid im Verhältnis von mindestens 5:1 steht, und das so erhältliche Dithiolbutandiol abtrennt.

Aus US 5,329,024 ist bekannt, dass man Olefine in Gegenwart von Mangan-Komplexen mit Hilfe von großen molaren Überschüssen an H₂O₂, beispielsweise Olefin : H₂O₂ wie 1:100) zu Epoxiden umsetzen kann.

Aus D. de Vos et al., Tetrahedron Lett. 1998, 39, 3221 ist bekannt, dass man Bisepoxide von Isopren und von 4-Vinyl-cyclohexen herstellen kann, indem man die betreffenden Diene mit einem großen Überschuss (Molverhältnis etwa 12:1) H₂O₂ in Gegenwart eines Mangan-Komplexes umsetzt. Trotz der großen Überschüsse an H₂O₂ fallen jedoch beträchtliche Anteile an Monoepoxiden an. Auch ist die Ausbeute an gewünschten Bisepoxiden noch verbesserungsfähig.

Es bestand also die Aufgabe, ein Verfahren bereit zu stellen, um Bisepoxide und Dithiole in guter Ausbeute und hinreichender Reinheit darzustellen. Es bestand ferner die Aufgabe, neue Gemische von Dithiolen und Verwendungen für Gemische von Dithiolen bereit zustellen.

Demgemäß wurde das eingangs definierte Verfahren gefunden.

Erfindungsgemäß setzt man ein konjugiertes Dien der allgemeinen Formel I um, 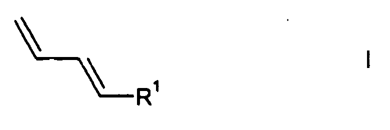 in dem
- R¹: ausgewählt aus
C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec.-Butyl und tert.-Butyl;
C₁-C₁₂-Alkyl, substituiert mit einer oder mehreren Hydroxy- oder Thiolgruppen wie Hydroxymethyl, 2-Hydroxyethyl, 1,2-Dihydroxyethyl, 3-Hydroxy-n-propyl, 2-Hydroxy-iso-Propyl, ω-Hydroxy-n-butyl, ω-Hydroxy-n-decyl, HS-CH₂-;
HS-(CH₂)₂- oder HS-(CH₂)₃-,
und ganz besonders bevorzugt Wasserstoff.

Natürlich kann man auch Mischungen von Olefinen oder Dienen umsetzen, die konjugiertes Dien der allgemeinen Formel I enthalten.

Erfindungsgemäß erfolgt die Umsetzung in Gegenwart von Katalysator, der erhältlich ist
durch Kontaktieren von mindestens einer Manganverbindung, gewählt aus A₂MnX₄, AMnX₃, MnY, MnX₂ und MnX₃
mit mindestens einem Liganden L der allgemeinen Formel II 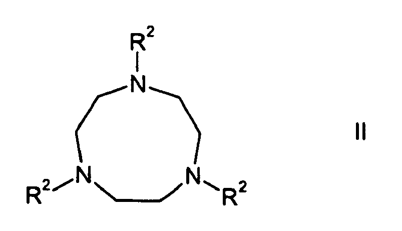 und mindestens einem Coliganden, der von Monocarbonsäuren, zwei- oder mehrwertigen Carbonsäuren oder Diaminen abgeleitet ist,
wobei die Variablen wie folgt definiert sind:
- X: verschieden oder gleich und gewählt aus einwertigen Anionen, R³O⁻, F⁻, Cl⁻, Br⁻, I⁻, NCS⁻, N₃⁻, I₃⁻, R³COO⁻, R³SO₃⁻, R³SO₄⁻, OH⁻, CN⁻, OCN⁻, NO₃⁻, ClO₄⁻, PF₆⁻, BPh₄⁻ mit Ph = Phenyl und F₃CSO₃⁻. Besonders bevorzugt sind Cl⁻ und Acetat.
- Y: ein zweiwertiges Anion, besonders bevorzugt SO₄²⁻ und HPO₄²⁻.
- A: verschieden oder vorzugsweise gleich und gewählt aus Alkalimetallkationen, beispielsweise Li⁺, Na⁺, K⁺, Rb⁺ und Cs⁺, insbesondere Na⁺ und K⁺
und Ammonium NH₄⁺, welches alkyliert sein kann, beispielsweise N(R⁴)(R⁵)(R⁶)(R⁷)⁺, wobei R⁴ bis R⁷ jeweils gleich oder verschieden sind und ausgewählt aus Wasserstoff, Benzyl, C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec.-Butyl und tert.-Butyl, Phenyl oder CH₂-CH₂-OH. Beispielhaft seien Tetramethylammonium, Tetraethylammonium, Methyldiethanolammonium und n-Butyldiethanolammonium genannt.
- R²: verschieden oder vorzugsweise gleich und gewählt aus verzweigtem oder vorzugsweise unverzweigtem C₁-C₂₀-Alkyl wie beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Octyl, n-Decyl, n- Dodecyl, n-Tetradecyl, n-Hexadecyl, n-Octadecyl und n-Eicosyl, bevorzugt unverzweigtes C₁-C₁₂-Alkyl wie Methyl, Ethyl, n-Propyl, n-Butyl, n-Pentyl, n-Hexyl, n-Heptyl, n-Octyl, n-Decyl oder n-Dodecyl, besonders bevorzugt C₁-C₄-Alkyl wie Methyl, Ethyl, n-Propyl, n-Butyl, und ganz besonders bevorzugt Methyl.
- R³: steht vorzugsweise für
- C₁-C₂₀-Alkyl,: beispielsweise Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Octyl, n-Decyl, n- Dodecyl, n-Tetradecyl, n-Hexadecyl, n-Octadecyl und n-Eicosyl, bevorzugt unverzweigtes C₁-C₁₂-Alkyl wie Methyl, Ethyl, n-Propyl, n-Butyl, n-Pentyl, n-Hexyl, n-Heptyl, n-Octyl, n-Decyl oder n-Dodecyl, besonders bevorzugt C₁-C₄-Alkyl wie Methyl, Ethyl, n-Propyl, n-Butyl, und ganz besonders bevorzugt Methyl,
- substituiertes: C₁-C₂₀-Alkyl wie beispielsweise ω-Cyclohexylpropyl, 2-Cyclohexylethyl;
- C₃-C₁₂-Cycloalkyl: wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl und Cycloheptyl,
- C₆-C₁₄-Aryl,: beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl
oder Benzyl.

Besonders bevorzugte Beispiele für erfindungsgemäß eingesetzte Manganverbindungen sind Mangan(II)sulfat, Mangan(II)acetat, Mangan(II)chlorid, Mangan(II)perchlorat oder Kaliumhexachloromanganat(IV) K₂MnCl₆.

Dabei ist es möglich, dass erfindungsgemäß eingesetzte Manganverbindungen Kristallwasser und/oder Hydratwasser aufweisen wie beispielsweise Mn(OAc)₂·4 H₂O, MnSO₄·H₂O, Mn(ClO₄)₂·6 H₂O, MnCl₂·4 H₂O.

In einer Ausführungsform der vorliegenden Erfindung setzt man im Bereich von 0,001 bis 0,1, besonders bevorzugt 0,005 bis 0,01 Äquivalente Manganverbindung ein, bezogen auf Dien der allgemeinen Formel I.

In einer anderen Ausführungsform der vorliegenden Erfindung setzt man im Bereich von 0,00001 bis 0,001, besonders bevorzugt 0,0001 bis 0,0005 Äquivalente Manganverbindung ein, bezogen auf Dien der allgemeinen Formel I.

In einer Ausführungsform der vorliegenden Erfindung setzt man 1 bis 5 Äquivalente Ligand L der allgemeinen Formel II, bezogen auf Mangan, ein, bevorzugt 1,1 bis 2 Äquivalente.

Als Coliganden sind solche Verbindungen geeignet, die von Monocarbonsäuren, zwei- oder mehrwertigen Carbonsäuren oder Diaminen abgeleitet sind, d.h. Monocarbonsäuren, zwei- oder mehrwertigen Carbonsäuren und Diamine selbst sowie im Falle von Monocarbonsäuren, zwei- oder mehrwertigen Carbonsäuren insbesondere deren korrespondierende Alkalimetallsalze.

In einer Ausführungsform der vorliegenden Erfindung sind Coliganden von solchen Monocarbonsäuren bzw. zwei- oder mehrwertigen Carbonsäuren abgeleitet, deren pKₐ-Wert bzw. pKₐ¹-Wert in Wasser bei 25°C unter 7 liegt.

In einer Ausführungsform der vorliegenden Erfindung sind Coliganden abgeleitet von Oxalsäure (III.1), Dihydroxyfumarsäure (III.2), Weinsäure (III.3), Maleinsäure (III.4), Quadratsäure (III.5), 2-Sulfobenzoesäure (III.6) und N(p-Toluolsulfonyl)glycin (III.7):

HOOC-COOH III.1

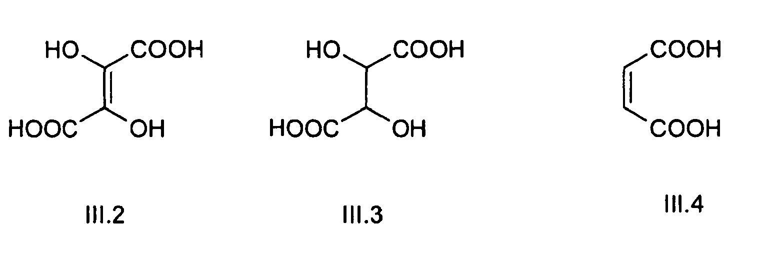

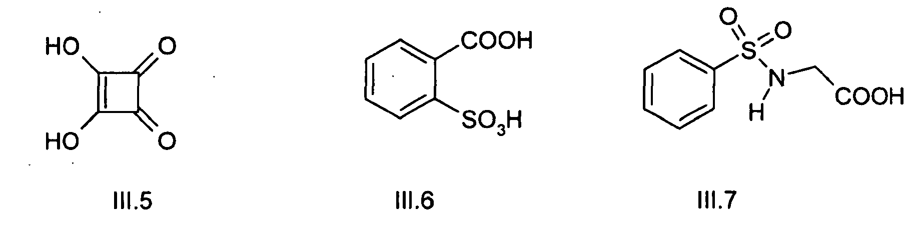

Weiterhin ist Ascorbinsäure geeignet.

Ein weiterer ganz besonders bevorzugter Coligand ist 1,2-Diaminocyclohexan, wobei sowohl das Isomerengemisch als auch die jeweiligen cis- und trans-Isomeren in angereicherter Form in Frage kommen.

In einer Ausführungsform der vorliegenden Erfindung setzt man Coliganden als Mischung von Monocarbonsäuren und Alkalimetallsalz der betreffenden Monocarbonsäure ein.

In einer Ausführungsform der vorliegenden Erfindung setzt man Coliganden als Mischung von zwei- oder mehrwertiger Carbonsäure und Alkalimetallsalz der betreffenden zwei- oder mehrwertigen Carbonsäure ein.

In einer Ausführungsform der vorliegenden Erfindung setzt man 0,1 bis 5 Äquivalente, bevorzugt 0,5 bis 1 Äquivalent Coligand ein, bezogen auf Mangan.

Erfindungsgemäß setzt man Dien der allgemeinen Formel I mit mindestens einem Peroxid um, wobei man bis zu 4 Äquivalenten Peroxid pro Äquivalent C-C-Doppelbindung einsetzt. Vorzugsweise setzt man mindestens ein Äquivalent Peroxid pro Äquivalent C-C-Doppelbindung ein. Als Peroxid bevorzugt organische Peroxide einsetzen, insbesondere tert.-Butylhydroperoxid, Cumolhydroperoxid, 1,3-Diisopropylmonohydroperoxid, 1-Phenyl-ethylhydroperoxid. Wasserstoffperoxid (H₂O₂) ist als Peroxid besonders bevorzugt.

Wünscht man Wasserstoffperoxid einzusetzen, so setzt man es als wässrige Lösung ein, beispielsweise als 30 Gew.-% oder 50 Gew.-% Lösung, deren Gehalt an reaktionsfähigem H₂O₂ man nach sich bekannten Methoden, beispielsweise durch Titration, ermitteln kann.

In einer Ausführungsform der vorliegenden Erfindung setzt man bis zu 3, bevorzugt bis zu 2,1 Äquivalenten Peroxid pro Äquivalent C-C-Doppelbindung ein.

Für die Reihenfolge zur Kontaktierung der Reaktionspartner des erfindungsgemäßen Verfahrens sind mehrere Vorgehensweisen möglich.

In einer Ausführungsform der vorliegenden Erfindung mischt man zunächst Ligand L der allgemeinen Formel II und Coligand mit Dien der allgemeinen Formel I und Peroxid und setzt anschließend Manganverbindung zu.

In einer anderen Ausführungsform der vorliegenden Erfindung mischt man zunächst Ligand L der allgemeinen Formel II mit Coligand und Dien der allgemeinen Formel I und Manganverbindung und setzt anschließend Peroxid zu.

In einer anderen Ausführungsform der vorliegenden Verbindung stellt man zunächst durch Kontaktieren von Manganverbindung und Ligand L und Coligand der allgemeinen Formel II eine Komplexverbindung her, die man anschließend mit Dien der allgemeinen Formel I und danach mit Peroxid mischt.

In einer anderen Ausführungsform der vorliegenden Verbindung stellt man zunächst durch Kontaktieren von Manganverbindung und Ligand L der allgemeinen Formel II eine Komplexverbindung her, die man anschließend mit Dien der allgemeinen Formel I und Coligand und danach mit Peroxid mischt.

In einer anderen Ausführungsform der vorliegenden Verbindung stellt man zunächst durch Kontaktieren von Manganverbindung und Ligand L der allgemeinen Formel II eine Komplexverbindung der Formel [LMn(µ-O)₃MnL]X her, die man anschließend mit Dien der allgemeinen Formel I und Coligand und danach mit Peroxid mischt.

In einer anderen Ausführungsform der vorliegenden Verbindung stellt man zunächst durch Kontaktieren von Manganverbindung und Ligand L und Coligand der allgemeinen Formel II eine Komplexverbindung her, die man anschließend mit Dien der allgemeinen Formel I und danach mit Peroxid mischt, wobei man Peroxid in zwei Portionen in einem Zeitabstand von mindestens 2 Stunden zugibt.
Es ist nicht genau bekannt, in welcher Form die katalytisch aktive Spezies vorliegt. Ohne einer Theorie den Vorzug geben zu wollen, erscheint es denkbar, dass Mangan während der katalytischen Reaktion zumindest zeitweise in der Oxidationsstufe +IV vorliegt. Weiterhin erscheint es möglich, dass während der katalytischen Reaktion zumindest zeitweise einfach oder mehrfach µ-Oxo-verbrückte Spezies vorliegen.

In einer Ausführungsform der vorliegenden Erfindung führt man das erfindungsgemäße Verfahren in einem Lösungsmittel oder einem Gemisch von Lösungsmitteln durch. Als Lösungsmittel kann man bei Reaktionstemperatur flüssige organische oder anorganische Flüssigkeiten verwenden, die unter den Bedingungen nicht oder nur in vernachlässigbaren Anteilen mit den Reaktionspartnern und Produkt, d.h. beispielsweise Bisepoxid, reagieren.

Geeignet sind beispielsweise C₁-C₄-Alkanole wie Methanol, Ethanol, n-Propanol, isoPropanol, weiterhin Ketone wie beispielsweise Aceton, Methylethylketon und Methylisobutylketon (MIBK), Acetonitril, halogenierte Kohlenwasserstoff wie beispielsweise Methylenchlorid, Chloroform, 1,1,2,2-Tetrachlorethan und Wasser. Besonders gut geeignet sind Gemische aus Wasser und Acetonitril, Gemische aus Wasser und Methanol und Gemische aus Wasser und Aceton.

In einer Ausführungsform der vorliegenden Erfindung arbeitet man mit so viel Lösungsmittel bzw. Gemisch von Lösungsmitteln, dass die Konzentration von Bisepoxid 50 Gew.-% nicht überschreitet, bevorzugt 5 bis 15 Gew.-%.

In einer Ausführungsform der vorliegenden Erfindung führt man das erfindungsgemäße Verfahren durch, ohne den Katalysator vorher auf einem oder mehreren festen Trägermaterialien wie beispielsweise Kieselgel oder Aluminiumoxid immobilisiert zu haben.

In einer Ausführungsform der vorliegenden Erfindung führt man das erfindungsgemäße Verfahren bei Temperaturen im Bereich von -50 bis 100°C, bevorzugt von -30 bis 80 °C, besonders bevorzugt von -10 bis 60 °C und ganz besonders bevorzugt von 0 bis 5°C durch.

In einer Ausführungsform der vorliegenden Erfindung führt man das erfindungsgemäße Verfahren bei einem Druck im Bereich von 1 bis 200 bar, bevorzugt bei 1 bis 100 bar, besonders bevorzugt bei Normaldruck bis 10 bar durch.

In einer Ausführungsform der vorliegenden Erfindung führt man das erfindungsgemäße Verfahren bei einem pH-Wert von 1 bis 7, bevorzugt von 3 bis 5 bis durch.

In einer Ausführungsform der vorliegenden Erfindung beträgt die Reaktionsdauer 1 Minute bis 24 Stunden, bevorzugt 30 Minuten bis 20 Stunden.

Als Reaktionsgefäße für die Ausübung des erfindungsgemäßen Verfahrens sind im Prinzip alle gängigen Reaktionsgefäße geeignet, beispielsweise Rohrreaktoren und Rührkessel, wobei man Rührkessel absatzweise oder kontinuierlich betreiben kann und Rohrreaktoren vorzugsweise kontinuierlich.

Durch das erfindungsgemäße Verfahren erhält man Lösungen von Bisepoxid. Die erfindungsgemäß erhältlichen Lösungen von Bisepoxid können geringe Anteile an Monoepoxid, beispielsweise der Formel V.1 oder V.2, 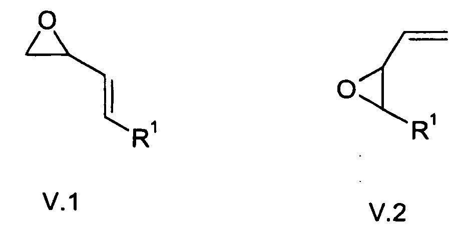 enthalten, wobei der Anteil an Monoepoxid in der Regel unter 8 mol-%, bezogen auf reines Bisepoxid, beträgt. Man kann Bisepoxid aus den erfindungsgemäß erhältlichen Lösungen isolieren und aufreinigen.

Ein weiterer Gegenstand der vorliegenden Erfindung ist ein Verfahren zur Herstellung von Dithiolgemischen, im Folgenden auch als erfindungsgemäßes Zweistufenverfahren bezeichnet, dadurch gekennzeichnet, dass man
(a) in einer ersten Stufe Bisepoxid nach einem Verfahren wie vorstehend beschrieben herstellt und
(b) ohne Isolierung von in Stufe (a) hergestelltem Bisepoxid in Gegenwart mindestens eines basischen Katalysators mit H₂S umsetzt.

In einer Ausführungsform der vorliegenden Erfindung setzt man Lösungen von Bisepoxid ein, welche nach einem vorstehend beschriebnen Verfahren erhältlich sind, und verzichtet auf Isolierungs- und Aufreinigungsoperationen.

In einer Ausführungsform setzt man in Stufe (b) mit 1 bis 10 Äquivalenten H₂S um, bevorzugt mit 1 bis 2 Äquivalenten H₂S, bezogen auf Äquivalent Epoxidgruppe.

Stufe (b) des erfindungsgemäßen Zweistufenverfahrens übt man in Gegenwart mindestens eines basischen Katalysators aus.

Als basische Katalysatoren sind basische Alkalimetallsalze und Ammoniumsalze geeignet, beispielsweise Alkalimetallhydroxide, Alkalimetallcarbonate, Alkalimetallhydrogensulfide, Ammoniumhydroxide. Als Alkalimetallkationen sind beispielsweise Li⁺, Na⁺, K⁺, Rb⁺ und Cs⁺, insbesondere Na⁺ und K⁺ zu nennen.

Als Ammoniumionen sind nicht nur unsubstituiertes NH₄⁺, zu nennen, sondern auch ein- und bis zu vierfach alkyliertes Ammonium zu nennen, beispielsweise N(R⁴)(R⁵)(R⁶)(R⁷)⁺, wobei R⁴ bis R⁷ jeweils gleich oder verschieden sind und ausgewählt aus Wasserstoff, Benzyl, C₁-C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl oder n-Decyl, besonders bevorzugt C₁-C₄-Alkyl wie Methyl, Ethyl, n-Propyl, iso-Propyl, n-Butyl, iso-Butyl, sec.-Butyl und tert.-Butyl, Phenyl oder CH₂-CH₂-OH. Beispielhaft seien Tetramethylammonium, Tetraethylammonium, Benzyltrimethylammonium, Methyldiethanolammoniunium und n-Butyldiethanolammonium genannt.

Bevorzugt ist mindestens ein basischer Katalysator in Stufe (b) gewählt aus Alkalimetallhydrogensulfid, Alkalimetallhydroxid und Benzyltri(C₁-C₁₀-Alkyl)ammoniumhydroxid, ganz besonders bevorzugt sind Natriumhydrogensulfid, Kaliumhydrogensulfid, Natriumhydroxid, Kaliumhydroxid und Benzyltrimethylammoniumhydroxid.

In einer Ausführungsform der vorliegenden Erfindung setzt man im Bereich von 10⁻⁴ bis 10 Gew.%, bevorzugt 0,5 bis 5 Gew.-% basischen Katalysator ein, bezogen auf Bisepoxid.

In einer Ausführungsform der vorliegenden Erfindung führt man Stufe (b) bei einem Druck im Bereich von 1 bis 200 bar, bevorzugt bei 1 bis 100 bar, besonders bevorzugt bei 1 bis 10 bar aus.

In einer Ausführungsform der vorliegenden Erfindung führt man Stufe (b) bei einer Temperatur im Bereich von - 50 bis 100 °C, bevorzugt von -30 bis 80 °C, besonders bevorzugt von -10 bis 60 °C, ganz besonders bevorzugt von 15 bis 35 °C aus.

In einer Ausführungsform der vorliegenden Erfindung führt man das erfindungsgemäße Verfahren bei einem pH-Wert von 8 bis 13, bevorzugt von 9 bis 11 durch.

In einer Ausführungsform der vorliegenden Erfindung geht man von einer nach Stufe (a) des erfindungsgemäßen Verfahrens erhältlichen Lösung von Bisepoxid aus, gibt H₂S zu, gibt danach mindestens einen basischen Katalysator zu und lässt reagieren.

In einer Ausführungsform der vorliegenden Erfindung kann man in Stufe (b) des erfindungsgemäßen Zweistufenverfahrens weiteres Lösungsmittel, gewählt aus den oben unter Stufe (a) aufgeführten Lösungsmitteln, zugeben.

In einer Ausführungsform der vorliegenden Erfindung beträgt die Reaktionsdauer 10 min bis 4 h, besonders bevorzugt 0,5 Stunden bis 2 Stunden.

Als Reaktionsgefäße für die Ausübung des erfindungsgemäßen Zweistufenverfahrens sind im Prinzip alle gängigen Reaktionsgefäße geeignet, beispielsweise Rohrreaktoren und Rührkessel, wobei man Rührkessel absatzweise oder kontinuierlich betreiben kann und Rohrreaktoren vorzugsweise kontinuierlich. Auch kontinuierlich betriebene Rührkesselkaskaden sind als geeignete Gefäße denkbar.

Ohne einer Theorie den Vorzug geben zu wollen, erscheint es denkbar, dass durch gegebenenfalls überschüssiges H₂S in Stufe (b) nicht abreagiertes Peroxid aus Stufe (a) abgefangen wird.

Durch Ausübung des erfindungsgemäßen Zweistufenverfahrens erhält man Lösungen von Dithiolgemischen bzw. korrespondierenden Salzen von Dithiolen, die ebenfalls ein Gegenstand der vorliegenden Erfindung sind. Aus erfindungsgemäßen Lösungen von Dithiolgemischen lassen sich Dithiolgemische bzw. ihre korrespondierenden Salze nach an sich bekannten Methoden isolieren, beispielsweise Neutralisieren, Abdestillieren des oder der Lösungsmittel. Um besonders reines Dithiolgemisch zu erhalten, kann man destillieren, beispielsweise unter vermindertem Druck.

Durch an sich bekannte Methoden wie beispielsweise Chromatographie kann man durch das erfindungsgemäße Verfahren erhältliche Dithiolgemische auftrennen in erythro- und threo-Dithiol, und durch chirale Diskriminierung kann man die Enantiomeren von threo-Dithiol trennen oder anreichern.

Die Erfindung wird durch Arbeitsbeispiele erläutert.
1. Herstellung eines Gemischs aus 40 mol-% erythro-1,4-Dimercaptobutan-2,3-diol und 60 mol-% racemischem threo-1,4-Dimercaptobutan-2,3-diol

### (a) Herstellung von Bisepoxid

### (a.1)

In einen 150-ml-Glasautoklaven mit Einlassrohr wurden miteinander vermischt:
42,6 g Acetonitril,
9 ml wässrige Mangan(II)-acetat-Lösung mit einer Konzentration von 0,02 mol Mn/l
9 ml 1,4,7-Trimethyl-1,4,7-triazacyclononan mit einer Konzentration von 0,03 mol/l
9 ml wässriger Natriumoxalat/Oxalsäurepuffer (Molverhältnis: 1:1) mit einer Konzentration von 0,06 mol/l aus Summe von Oxalat und Oxalsäure.

Die so erhältliche Lösung wurde mit Hilfe eines Trockeneis/Aceton-Bades auf etwa -40°C gekühlt. Anschließend wurden 3,34 g (61,8 mmol) 1,3-Butadien einkondensiert. Anschließend wurde mit Hilfe eines Eisbads eine Temperatur von 0°C eingestellt.

Anschließend wurden innerhalb von 1 Stunde 16,7 g 50 Gew.-% wässrige H₂O₂-Lösung (246 mmol H₂O₂) zugepumpt, wobei darauf geachtet wurde, dass die Temperatur nicht über 25°C stieg. Man beobachtete, dass der Druck im Autoklaven auf 5,2 bar stieg. Danach wurde das Eisbad entfernt und 2 Stunden bei Zimmertemperatur nachgerührt. Danach hatte sich ein Druck von 3,2 bar eingestellt.

Anschließend wurden 16,6 g 50 Gew.-% wässrige H₂O₂-Lösung (244 mmol H₂O₂) zugepumpt, wobei darauf geachtet wurde, dass die Temperatur nicht über 25°C stieg. Man beobachtete, dass der Druck im Autoklaven auf 3,8 bar stieg. Danach wurde das Eisbad entfernt und 5,5 Stunden bei Zimmertemperatur gerührt. Nach 5,5 Stunden hatte sich ein Druck von 5 bar eingestellt.
Anschließend wurde entspannt und die Zusammensetzung der resultierenden hellen Lösung (94,6 g) durch Gaschromatographie ermittelt. Man ermittelte einen Gehalt von 58,4 mmol Bisepoxid der Formel VII.1 und 3,4 mmol Vinyloxiran IV.1.1

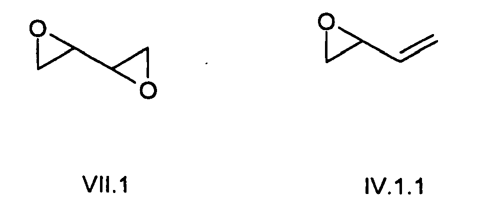

Die Ausbeute an gewünschtem Bisepoxid VII.1 betrug 95,5 %.

### (a.2)

Die Reaktion aus (a.1) wurde wiederholt, jedoch wurde nach dem Zupumpen von 16,7 g 50 Gew.-% wässriger H₂O₂-Lösung und Entfernung des Eisbads 12 Stunden bei Zimmertemperatur gerührt. Anschließend wurde wie unter (a.1) beschrieben weitergearbeitet. Man erhielt Bisepoxid VII.1 in sehr guter Ausbeute.

### (b) Herstellung von Dithiolgemisch

50 g der aus 1 (a.1) resultierenden Lösung wurden in einem 400-ml-Glasautoklaven vorgelegt und bei Zimmertemperatur mit 6 bar H₂S aufgepresst. Danach wurde eine Lösung von 1,04 g NaOH (fest) in 20 ml Methanol mit Hilfe einer HPLC-Pumpe zugesetzt. Während der Zugabe von Methanol/NaOH wurde eine Temperaturerhöhung von 25 auf 35 °C beobachtet.

Dabei wurde durch kontinuierliches Aufpressen von H₂S der Druck auf 6 bar gehalten. Danach wurden die Leitungen der HPLC-Pumpe mit 50 ml Acetonitril nachgespült.

Nach beendeter Reaktion, was sich durch Abklingen der Temperatur bemerkbar machte, wurde der Autoklav entspannt und über einen Zeitraum von 14 Stunden durch Durchleiten von N₂ durch die Reaktionsmischung von überschüssigem H₂S befreit.

Man erhielt 83,8 g einer klaren Lösung von Dithiolgemisch. Das erfindungsgemäß erhaltene Dithiolgemisch IV.1 wurde gaschromatographisch getrennt und hatte gemäß Gaschromatographie die folgende Zusammensetzung:
40 mol-% erythro-IV.1
60 mol-% threo-IV.1 als Racemat.
Bedingungen für die Gaschromatographie: Säule: HP-5, Länge: 30 m, Innendurchmesser= 0,25 mm, Filmdicke 0,25 µm, Detektor: WLD , Init. T.: 40°C, Init. Zeit: 5 min, Rate: 10°C/min, Finale Temperatur 290°C, Retentionszeit IV.1: 18,00-18,50 min.

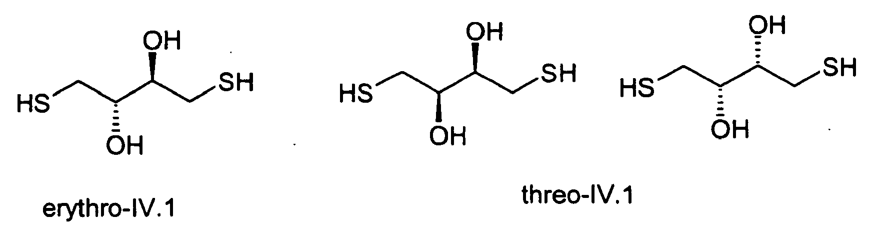

### 2. Behandlung von Blößen mit Dithiolgemisch IV. 1

Die Werte in Gew.-% beziehen sich jeweils auf das Salzgewicht, wenn nicht anderes angegeben ist.

### Allgemeine Vorbehandlung:

Die Haut eines Süddeutschen Rindes wurde zunächst bei 28°C mit 200 Gew.-% Wasser und 0,2 Gew.-% C₁₅H₃₁-O-(CH₂-CH₂-O)₇-H 10 Minuten in einem Fass bei leichtem Umrühren vorgeweicht. Die Flotte wurde abgelassen und danach mit 100 Gew.-% Wasser, 0,2 Gew.-% C₁₅H₃₁-O-(CH₂-CH₂-O)₇-H und 0,5 Gew.-% Na₂CO₃ bei gelegentlichem Rühren 19 Stunden eingeweicht. Anschließend wurde die Flotte abgelassen.

Die geweichten Häute süddeutscher Rinder wurden grün entfleischt (Stärke etwa 4 mm) und die Croupons der Häute in Hautstücke zu je 2,5 kg Grüngewicht geschnitten.

Im Folgenden beziehen sich die Werte in Gew.-% jeweils auf das Grüngewicht, wenn nicht anderes vermerkt.

### 2.1. Äscher des Vergleichsbeispiels V1

Für das Vergleichsbeispiel V1 wurden 100 Gew.-% Grüngewicht in einem drehbaren 10-I-Fass mit strömungsbrechenden Inneneinbauten nacheinander mit 60 Gewichtsteilen Wasser, 0,8 Gew.-% NaSH und 3 Gew.-% Kalkhydrat beaufschlagt. Es folgten im Abstand von 30 Minuten je 0,75 Gew.-% Natriumsulfid. Das Fass wurde weitere 45 Minuten bei 15 Umdrehungen/Minute betrieben. Anschließend wurden weitere 40 Gewichtsteile Wasser dosiert. Nach 10 Stunden bei 23 bis 27°C und 5 Umdrehungen/Minute wurden die Versuche beendet, indem die Flotte abgelassen wurde und die Haute zweimal 15 Minuten mit 150 Gewichtsteilen Wasser gewaschen wurden.

### 2.2. Haarzerstörender Äscher der Beispiele 2.1 bis 2.4

In den Beispielen 2.1 bis 2.4 wurden 100 Gew.-% Grüngewicht in drehbare 10-I-Fässer mit strömungsbrechenden Inneneinbauten zunächst mit
60 Gew.-% Wasser versetzt und anschließend wie aus Tabelle 1 ersichtlich mit Produkten beaufschlagt.

**Tabelle 1**

| Beispiel | Einsatzmenge [Gew.-%] | Produkt | Zeit [min] |
|---|---|---|---|
| 2.1 | 0,5 | Natriumsulfhydrat (70%) | |
| | 0,5 | Dithiolgemisch IV.1 | 60 |
| | 1,2 | Kalkhydrat | 60 |
| | 1,2 | Kalkhydrat | 60 |
| 2.2 | 1,0 | Dithiolgemisch IV.1 | 60 |
| | 1,2 | Kalkhydrat | |
| | 1,2 | Kalkhydrat | 60 |
| 2.3 | 1,5 | Dithiolgemisch IV.1 | 60 |
| | 1,2 | Kalkhydrat | 60 |
| | 1,2 | Kalkhydrat | 60 |
| 2.4 | 1,0 | Dithiolgemisch IV.1 | 60 |
| | 1,0 | Wässrige Natriumhydroxid-Lsg. (50 Gew.-%) | 30 |
| | 1,0 | Wässrige Natriumhydroxid-Lsg. (50 Gew.-%) | 30 |
| | 50 | Wasser | |
| | 0,4 | Wässrige Natriumhydroxid-Lsg. (50 Gew.-%) | 60 |
| | 50 | Wasser | 30 |

Die Fässer wurden weitere 45 Minuten bei 5 Umdrehungen/Minute betrieben. Anschließend wurden weitere 40 Gew.-% Wasser dosiert. Nach 10 Stunden bei 23 bis 27°C bei periodischem Betreiben mit 3 Umdrehungen/Minute über jeweils 5 Minuten pro Stunde wurden die Versuche beendet, indem die Flotten abgelassen wurden und die erhaltenen Blößen zweimal für je 15 Minuten mit 150 Gew.-% Wasser gewaschen wurden.

### 2.3. Beurteilung von Blößen gemäß Vergleichbeispiel und gemäß den Beispielen 2.1 bis 2.4 nach dem Äscher

Die gemäß den Beispielen 2.1 bis 2.4 behandelten Blößen waren den nach Vergleichsbeispiel V1 behandelten Häuten hinsichtlich der Schwellung nur wenig überlegen, zeichneten sich aber durch einen glatteren und flacheren Narben aus, insbesondere die Blößen der Beispiele 2.3 bis 2.4. Die Epidermis und die Haare mit Haarwurzel in den Blößen nach Beispiel 2.1 bis 2.3 waren weitgehend und in Blöße nach Beispiel 2.4 vollständig zerstört.

## Patentansprüche

1. Verfahren zur Herstellung von Bisepoxiden, **dadurch gekennzeichnet, dass** man ein konjugiertes Dien der allgemeinen Formel I 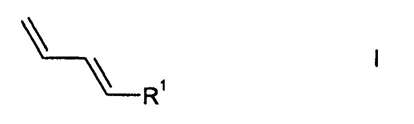 in dem
R¹ gewählt wird aus Wasserstoff und C₁-C₁₂-Alkyl, unsubstituiert oder substituiert mit einer oder mehreren S-H oder O-H-Gruppen,
in Gegenwart eines Katalysators, der erhältlich ist durch Kontaktieren von
mindestens einer Manganverbindung, gewählt aus A₂MnX₄, AMnX₃, MnY, MnX₂ und MnX₃
mit mindestens einem Liganden L der allgemeinen Formel II 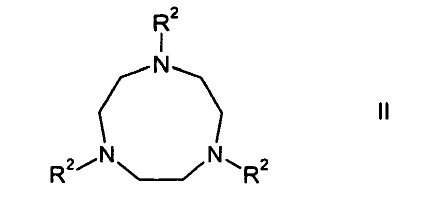
wobei die Variablen wie folgt definiert sind:
X verschieden oder gleich und gewählt aus einwertigen Anionen,
Y ein zweiwertiges Anion,
A gewählt aus Alkalimetall und Ammonium, welches alkyliert sein kann,
R² verschieden sind oder gleich und gewählt aus C₁-C₂₀-Alkyl, und mindestens einem Coliganden, der von Monocarbonsäuren, zwei- oder mehrwertigen Carbonsäuren oder Diaminen abgeleitet ist,
mit mindestens einem Peroxid umsetzt, wobei man bis zu 4 Äquivalenten Peroxid pro Äquivalent C-C-Doppelbindung einsetzt.

2. Verfahren nach Anspruch 1, **dadurch gekennzeichnet, dass** man es in einem Lösungsmittel oder einem Gemisch von Lösungsmitteln durchführt.

3. Verfahren nach Anspruch 1 oder 2, **dadurch gekennzeichnet, dass** der Coligand Oxalat ist.

4. Verfahren zur Herstellung von Dithiolgemischen, **dadurch gekennzeichnet, dass** man
(a) in einer ersten Stufe Bisepoxid nach einem Verfahren gemäß Anspruch 1 bis 3 herstellt und
(b) ohne Isolierung von in Stufe (a) hergestelltem Bisepoxid in Gegenwart mindestens eines basischen Katalysators mit H₂S umsetzt.

5. Verfahren nach Anspruch 4, **dadurch gekennzeichnet, dass** man mindestens einen basischen Katalysator in Stufe (b) wählt aus Alkalimetallhydrogensulfid, Alkalimetallhydroxid und Benzyltri-(C₁-C₁₀-alkyl)-ammoniumhydroxid.

## Claims

1. A process for the preparation of bisepoxides, wherein a conjugated diene of the formula I 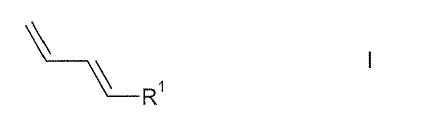 where
R¹ is selected from hydrogen and C₁-C₁₂-alkyl, unsubstituted or substituted by one or more S-H or O-H groups,
is reacted in the presence of a catalyst which is obtainable by bringing
at least one manganese compound selected from A₂MnX₄, AMnX₃, MnY, MnX₂ and MnX₃ into contact
with at least one ligand L of the formula II 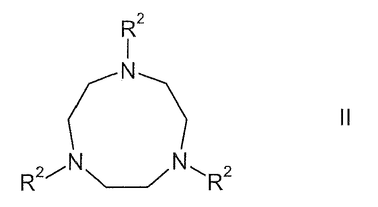
where
X are identical or different and are selected from monovalent anions,
Y is a divalent anion,
A is selected from alkali metal and ammonium, which may be alkylated,
R² are different or identical and are selected from C₁-C₂₀-alkyl, and at least one coligand which is derived from monocarboxylic acids, dibasic or polybasic carboxylic acids or diamines,
with at least one peroxide, up to 4 equivalents of peroxide being used per equivalent of C-C double bond.

2. The process according to claim 1, which is carried out in a solvent or a mixture of solvents.

3. The process according to claim 1 or 2, wherein the coligand is oxalate.

4. A process for the preparation of dithiol mixtures, wherein
(a) in a first stage, bisepoxide is prepared by a process according to any of claims 1 to 3 and
(b) is reacted with H₂S in the presence of at least one basic catalyst without isolation of bisepoxide prepared in stage (a).

5. The process according to claim 4, wherein at least one basic catalyst in stage (b) is selected from alkali metal hydrogen sulfide, alkali metal hydroxide and benzyltri(C₁-C₁₀-alkyl)ammonium hydroxide.

## Revendications

1. Procédé de fabrication de bis-époxydes, **caractérisé en ce que** l'on fait réagir un diène conjugué de formule générale I : 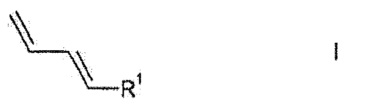 dans laquelle :
R¹ est choisi parmi l'hydrogène ou un groupement alkyle en C₁-C₁₂ non substitué ou substitué par un ou plusieurs groupements S-H ou O-H,
en présence d'un catalyseur, que l'on peut obtenir en mettant en contact
au moins un composé de manganèse, choisi parmi A₂MnX₄, AMnX₃, MnY, MnX₂ et MnX₃
avec au moins un ligand L de formule générale II 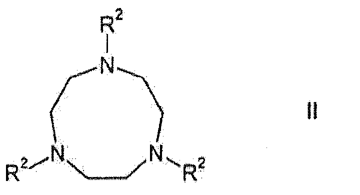
les variables étant définies de la manière suivante:
X représente des composés différents ou identiques et est choisi parmi des anions monovalents,
Y représente un anion divalent,
A est choisi parmi un métal alcalin et l'ammonium, lequel peut être alkylé,
R² représente des radicaux différents ou identiques et est choisi parmi un alkyle en C₁-C₂₀ et au moins un co-ligand, qui est dérivé d'acides mono-carboxyliques, d'acides carboxyliques di- ou polyvalents ou de diamines,
avec au moins un peroxyde, dans lequel on utilise jusqu'à 4 équivalents de peroxyde par équivalent de double liaison C-C.

2. Procédé selon la revendication 1, **caractérisé en ce que** celui-ci est effectué dans un solvant ou dans un mélange de solvants.

3. Procédé selon la revendication 1 ou 2, **caractérisé en ce que** le co-ligand est un oxalate.

4. Procédé de fabrication de mélanges de dithiols, **caractérisé en ce que**:
(a) on fabrique dans une première étape un bis-époxyde par un procédé selon les revendications 1 à 3, et
(b) on fait réagir le bis-époxyde fabriqué à l'étape (a), sans isolement de ce dernier, avec du H₂S en présence d'au moins un catalyseur basique.

5. Procédé selon la revendication 4, **caractérisé en ce que** l'on choisit au moins un catalyseur basique à l'étape (b) parmi un hydrogénosulfure de métal alcalin, un hydroxyde de métal alcalin et un hydroxyde de benzyltri-(alkyle en C₁-C₁₀))-ammonium.
